# EUROPEAN PATENT APPLICATION

(11) **EP 4 663 770 A1**
(43) Date of publication of application: **17.12.2025**
(21) Application number: 24753513.1
(22) Date of filing: 25.01.2024
(51) Int. Cl.: C12Q 1/683, C12Q 1/6818, C12Q 1/6886, C12Q 1/6848

(54) **METHOD FOR DETECTING MUTANT NUCLEIC ACID**

(30) Priority: 08.02.2023 KR 20230016723; 07.08.2023 KR 20230103139
(71) Applicant: Seoul National University R & DB Foundation, Seoul 08826 (KR)
(72) Inventor: SHIN, Soo Chul, Incheon 21561 (KR); HOHNG, Sung Chul, Seoul 06909 (KR)
(74) Representative: dompatent
(86) International application number: PCT/KR2024/001155
(87) International publication number: WO 2024/167181

(57) **Abstract**

The present invention relates to a method for detecting a mutant gene, wherein a nucleic acid targeting the mutant gene and its wild-type gene is used to increase detection speed and efficiency, reduce the false positive rate, and enable multiplex detection of low-concentration and low-frequency mutations.

## Description

### [Technical Field]

The present invention relates to a method for detecting mutant nucleic acids present at low concentrations and low frequencies.

### [Background Art]

The need to analyze low-concentration, low-frequency mutations is increasing in various fields such as oncology, organ transplantation, and infectious diseases. For example, tumor development is closely related to somatic mutations in cancer, and circulating tumor DNA (ctDNA) in the bloodstream has been proposed as a promising noninvasive biomarker in cancer management. Since ctDNA can serve as a reliable prognostic or predictive marker and can be used for cancer diagnosis and patient monitoring after treatment, the detection of low-concentration, low-frequency somatic mutations is becoming clinically important.

Diagnosing cancer using somatic mutations requires the analysis of multiple cancer-related genetic hotspots. In addition, in the early stage of cancer, the variant allele frequency (VAF) is extremely low, and the amount of ctDNA varies depending on the tumor size and cancer stage, thus reliable quantification of the VAF (%) is essential for early diagnosis. However, due to the excessive presence of wild-type cell-free DNA (cfDNA), it is difficult to accurately detect and quantify ctDNA.

Currently, next-generation sequencing (NGS) and digital PCR (dPCR) are widely used to detect low-frequency mutations, including single nucleotide variants (SNVs) and insertion/deletion (indel) mutations. NGS is a high-throughput technology that provides a comprehensive view of genetic mutations, but it has limitations such as low sensitivity (limit of detection, LoD: up to 1% VAF) and long processing time. Advanced approaches, such as the use of unique molecular identifiers (UMIs), improve sensitivity but incur significant additional costs. Meanwhile, dPCR offers better sensitivity (LoD: up to 0.1% VAF), but it is almost impossible to multiplex.

### [Summary of Invention]

### [Problems to be Solved by Invention]

It is an object of the present invention to provide a method for detecting mutant nucleic acids.

### [Means for Solving Problems]

1. A method for detecting a mutant nucleic acid, including the steps of: amplifying a first target nucleic acid present in a sample using a pair of primers including a first primer to which a phosphate group is bound and a second primer to which the phosphate group is not bound, to obtain an amplified product; treating the amplified product with a nuclease that specifically binds to the phosphate group to obtain a single-stranded first target nucleic acid to which the phosphate group is not bound; and fixing the single-stranded first target nucleic acid to a surface of a detection chip and then contacting it with a first probe labeled with a first fluorescent molecule.
2. The method for detecting a mutant nucleic acid according to the above 1, further including the step of detecting whether the first probe is bound to a mutation site of the single-stranded first target nucleic acid.
3. The method for detecting a mutant nucleic acid according to the above 1, further including the step of converting the remaining region of the single-stranded first target nucleic acid, excluding the binding region of the first probe, into a double-stranded region, before fixing the single-stranded first target nucleic acid to the surface of the detection chip.
4. The method for detecting a mutant nucleic acid according to the above 1, wherein the second primer to which the phosphate group is not bound is a second primer bound to biotin or digoxigenin.
5. The method for detecting a mutant nucleic acid according to the above 1, wherein the single-stranded first target nucleic acid is fixed to the surface of the detection chip by a complementary nucleic acid.
6. The method for detecting a mutant nucleic acid according to the above 5, wherein the complementary nucleic acid used for fixing to the surface of the detection chip is a nucleic acid bound to biotin or digoxigenin.
7. The method for detecting a mutant nucleic acid according to the above 1, wherein the sample is any one selected from the group consisting of blood, plasma, serum, urine, feces, cerebrospinal fluid, lung lavage fluid, pleural effusion, ascites, saliva, sputum, body fluid, tissue, and cells isolated from an individual.
8. The method for detecting a mutant nucleic acid according to the above 1, wherein the nucleic acid is DNA or RNA.
9. The method for detecting a mutant nucleic acid according to the above 1, wherein the nuclease is lambda exonuclease.
10. The method for detecting a mutant nucleic acid according to the above 1, wherein the surface of the detection chip is coated with avidin or anti-digoxigenin.
11. The method for detecting a mutant nucleic acid according to the above 1, further including the step of reverse transcribing RNA before amplifying the first target nucleic acid.
12. The method for detecting a mutant nucleic acid according to the above 1, further including the step of pretreating the nucleic acid to detect methylation thereof before amplifying the first target nucleic acid.
13. The method for detecting a mutant nucleic acid according to the above 1, wherein the first probe is conjugated to an Argonaute protein.
14. The method for detecting a mutant nucleic acid according to the above 2, wherein the detection is performed by measuring a FRET (Fluorescence Resonance Energy Transfer) signal.
15. The method for detecting a mutant nucleic acid according to the above 1, further including the step of amplifying a second target nucleic acid present in the sample using a pair of primers including a third primer to which a phosphate group is bound and a fourth primer to which the phosphate group is not bound.
16. The method for detecting a mutant nucleic acid according to the above 15, further including the step of fixing a single-stranded second target nucleic acid to which the phosphate group is not bound to the surface of the detection chip, and then contacting it with a third probe labeled with a third fluorescent molecule to perform multi-color imaging.
17. The method for detecting a mutant nucleic acid according to the above 1, further including the step of treating a second probe that complementarily binds to a wild-type nucleic acid and is not fluorescently labeled, together with the first probe.

### [Advantageous effects]

The mutation detection method of the present invention may reduce the false positive rate by including a probe targeting a wild-type nucleic acid that is not fluorescently labeled, thereby increasing the efficiency of mutant gene detection.

The mutation detection method of the present invention may be performed by replacing the probe to detect various mutant genes, thereby enabling the detection of multiple mutations within a single sample.

The mutation detection method of the present invention may detect a mutant target nucleic acid with high efficiency even when the concentration of the target nucleic acid to be detected is low, by including a predetermined amplification step.

### [Brief Description of Drawings]

FIG. 1 shows the oligonucleotide and primer sequences used in the present invention (p: phosphate group, [t]: amine-modified thymine).
FIG. 2 is a schematic diagram illustrating the experimental process referred to as SIMUL (single-molecule detection of multiple low-frequency mutations).
FIG. 3 shows the sequences of KRAS and KRAS G12V.
FIG. 4 shows the probe sequences of wild-type and G12V.
FIG. 5 shows the single-molecule fluorescence spots of the G12V probe.
FIG. 6 shows the linear relationship between the number of Cy-7 spots and VAF.
FIG. 7 shows the time trace results of Cy7 fluorescence identified as off-target (top) and on-target (bottom) by the detection probe using a 20% duty cycle criterion.
FIG. 8 shows the linear relationship between the number of spots and VAF, with a LoD measured at 0.008% VAF.
FIG. 9 shows the number of Cy7 spots in a 0.5-second single-frame snapshot at various VAFs of KRAS G12V mutations ranging from 0 to 0.05%.
FIG. 10 shows the sequences and probes (a-c) and the corresponding SIMUL results (d-f) for KRAS G12D, G12C, and G12V, respectively.
FIG. 11 shows the results of verifying the orthogonality of the probes.
FIG. 12 shows the results of detecting low-frequency mutations of G12D, G12C, and G12V.
FIG. 13 shows the results of detecting BRAF mutations V600K and V600E.
FIG. 14 shows the results of multiplex detection experiments for low-frequency mutations.
FIG. 15 and 16 show the results of detecting ctDNA mutations in plasma from healthy donors and lung cancer patients.

### [Mode for Carrying out Invention]

The present invention provides a method for detecting a mutant nucleic acid, including the steps of: amplifying a first target nucleic acid present in a sample using a pair of primers including a first primer to which a phosphate group is bound and a second primer to which the phosphate group is not bound, to obtain an amplified product; treating the amplified product with a nuclease that specifically binds to the phosphate group to obtain a single-stranded first target nucleic acid to which the phosphate group is not bound; and fixing the single-stranded first target nucleic acid to a surface of a detection chip and then contacting it with a first probe labeled with a first fluorescent molecule.

The detection method of the present invention performs predetermined amplification and single-stranded nucleic acid obtaining step prior to contacting the sample and the probe set, thereby enabling efficient detection of mutations even when the target nucleic acid (i.e., the mutant nucleic acid to be detected) is present at a low concentration.

When the concentration of the target nucleic acid in the sample is significantly low, the amplification step may be performed repeatedly to increase detection efficiency.

The sample may be at least one selected from the group consisting of blood, plasma, urine, cerebrospinal fluid, pleural effusion, ascites, saliva, sputum, body fluid, tissue, and cells isolated from an individual, but is not limited thereto.

The nucleic acid may be DNA or RNA, and the DNA may be circulating tumor DNA (ctDNA) or genomic DNA (gDNA), but is not limited thereto.

ctDNA is a genetic fragment derived from cancer cells contained within cell free DNA (cfDNA). cfDNA includes genetic fragments of various lengths present in the blood. In the case of healthy individuals, cfDNA is primarily derived from the DNA released from hematopoietic cells, whereas in the case of cancer patients, it includes ctDNA derived from cancer cells due to the death of cancer cells, as described below. It accounts for less than 0.1 to 10% of the total cfDNA. Since ctDNA contains genetic mutations related to specific cancers, monitoring these genetic mutations using blood may be useful for: early detection of cancer before lesion growth, analysis of responses to specific cancer treatments, discovery of mechanisms of resistance to anticancer drugs, and detection of the presence of residual cancer.

The method for detecting a mutant nucleic acid of the present invention may detect point mutations occurring in a nucleic acid, as well as mutation sites such as insertions, deletions, and translocations of genes.

In the present invention, the nucleic acid refers to various types of genes, such as coding genes, noncoding genes, cancer-related genes, and immune-related genes. In the case of cancer-related genes, examples may include KRAS, HRAS, NRAS, BRAF, APC, BCL2, BRCA1, BRCA2, ERBB2, MYC, RB1, and TP53, etc., but are not limited thereto.

The method for detecting a mutant nucleic acid of the present invention includes the step of amplifying a first target nucleic acid present in a sample using a pair of primers including a first primer to which a phosphate group is bound and a second primer to which the phosphate group is not bound, to obtain an amplified product.

The first primer and the second primer are a primer set (pair) required for amplifying the target nucleic acid.

A phosphate group (PO₃⁻) is bound to the 5' end of the first primer. The phosphate group specifically reacts with a nuclease. That is, the phosphate group allows the nuclease to bind to the nucleic acid to which a phosphate group is bound, thereby enabling the degradation or digestion of the nucleic acid. Accordingly, only the first target nucleic acid to which a phosphate group is not bound can be obtained.

The second primer is not bound to a phosphate group.

The second primer may consist solely of a sequence complementary to the first primer.

The second primer may be a nucleic acid having a chemical substance bound to a sequence complementary to the first primer, which allows immobilization of the single-stranded first target nucleic acid to the surface of a detection chip. The surface of the detection chip may be coated with a substance capable of specifically binding to the chemical substance, thereby enabling immobilization of the single-stranded first target nucleic acid to the surface of the detection chip. Examples of the chemical substance include biotin, digoxigenin, and the like, but are not limited thereto.

The method for detecting a mutant nucleic acid of the present invention includes the step of treating the amplified product with a nuclease that specifically binds to the phosphate group to obtain a single-stranded first target nucleic acid to which the phosphate group is not bound.

The nuclease is not limited to a specific type as long as it can specifically bind to a phosphate group. For example, the nuclease may be lambda exonuclease.

According to this step, the nucleic acid strand to which a phosphate group is bound among the amplified products is decomposed and eliminated, and only the single-stranded first target nucleic acid to which a phosphate group is not bound is obtained.

The method for detecting a mutant nucleic acid of the present invention includes the step of fixing the single-stranded first target nucleic acid to which a phosphate group is not bound to the detection chip surface, and then contacting it with a first probe labeled with a first fluorescent molecule.

When the first probe is contacted with the fixed single-stranded target nucleic acid, the first probe binds to the mutation site of the target nucleic acid, and the mutation may be detected using single-molecule fluorescence technology.

The first probe is labeled with a first fluorescent molecule, and thus emits fluorescence when it binds to the target mutant nucleic acid.

The first probe complementarily binds to the mutation site of the target nucleic acid. The first probe is labeled with a first fluorescent molecule. The first probe may be conjugated to an Argonaute protein to increase binding kinetics and enhance specificity and sensitivity.

The single-stranded first target nucleic acid may be fixed to the detection chip surface by a complementary nucleic acid coated on the detection chip surface. The complementary nucleic acid coated on the detection chip surface may be a nucleic acid consisting of a sequence complementary to the single-stranded first target nucleic acid, and may also be a nucleic acid consisting of a sequence complementary to the single-stranded first target nucleic acid labeled with biotin or digoxigenin.

If biotin, digoxigenin, or the like is bound to the single-stranded first target nucleic acid, a substance that can specifically bind to them may be coated on the detection chip surface to immobilize the single-stranded first target nucleic acid to the detection chip surface.

Fixing the single-stranded target nucleic acid to the detection chip surface offers the advantage of enabling effective measurement of single-molecule fluorescence signals. In addition, since the target nucleic acid is fixed, there is an advantage in that the probe and buffer used for detection may be easily and continuously changed.

The detection chip (chamber) may be coated with a substance that can bind to biotin, digoxigenin, etc. The coating material is not limited to a specific substance, as long as it can specifically bind to biotin, etc. The coating material may be, for example, avidin, anti-digoxigenin, etc. Avidin includes streptavidin, neutravidin, etc.

A second probe that complementarily binds to the wild-type nucleic acid may be used together with the first probe. The wild-type nucleic acid targeted by the second probe is the wild-type of the mutant nucleic acid targeted by the first probe. The second probe, like the first probe, may be conjugated to an Argonaute protein.

By contacting both the first probe and the second probe with the nucleic acid sample fixed to the detection chip, it is possible to prevent the first probe targeting the mutant nucleic acid from binding to the wild-type nucleic acid, thereby reducing the false positive rate of detection.

The method for detecting the mutant nucleic acid of the present invention may further include the step of treating a second probe that complementarily binds to a wild-type nucleic acid and is not fluorescently labeled, together with the first probe.

The method for detecting the mutant nucleic acid of the present invention may further include the step of detecting whether the first probe is bound to a mutation site of the single-stranded first target nucleic acid.

The detection method is not limited, and methods commonly used in the art may be utilized.

The detection method may be a method based on the fluorescence resonance energy transfer (FRET) phenomenon. For example, the method described in Korean Patent No. 10-2169649 may be applied to the present invention to detect whether the first probe is bound to the mutation site of the single-stranded first target nucleic acid.

The first probe of the present invention may be a FRET donor molecule or a FRET acceptor molecule. For example, the first probe may serve as a FRET donor molecule and be bound to the first target nucleic acid together with the FRET acceptor molecule, so that a FRET signal may be detected when both the first probe and the FRET acceptor molecule are bound to the first target nucleic acid, thereby determining whether the first probe is bound to the mutation site of the first target nucleic acid.

The method for detecting a mutant nucleic acid of the present invention may further include the step of converting the remaining region of the single-stranded first target nucleic acid, excluding the binding region of the first probe, into a double-stranded region, before fixing the single-stranded first target nucleic acid to the surface of the detection chip. By further including this step, it is possible to prevent the target nucleic acid from forming a secondary structure, which interferes with binding to the probe. This step is particularly necessary when the length of the target nucleic acid is long.

A complementary polynucleotide strand is bound to a region of the target nucleic acid other than the first probe binding site (the first probe non-binding site), so that the first probe non-binding site becomes double-stranded. Accordingly, the target nucleic acid may bind more effectively to the first probe.

The first probe binding site refers to a contiguous base sequence region including the mutation site of the target nucleic acid. The first probe binding site may include, for example, 5 to 15 consecutive bases.

The first probe may target a plurality of different mutation sites of the same gene. For example, when the gene is KRAS, the first probe may target KRAS G12C, G12D or G12V, and when the gene is BRAF, the first probe may target BRAF V600K or V600E. Probes targeting different mutation sites of the same gene as described above are each labeled with different fluorescent molecules, allowing the identification and quantification of the presence and frequency of each mutant variant.

In the present invention, complementary binding refers to the probe that can bind to the target base site under appropriate hybridization conditions to form a duplex, and includes a sequence with at least about 75%, at least about 80%, at least about 85%, at least about 90%, at least about 95%, or about 100% complementary sequence with the probe binding site.

The first probe includes a linker, and the linker includes an amine group. The first fluorescent molecule may be labeled to the first probe via the amine group of the linker. The first fluorescent molecule may be conjugated to either end (the 5' or 3' position of the nucleic acid) or an internal position of the probe.

The first fluorescent molecule may be, fluorescein, fluorescein chlorotriazinyl, rhodamine green, rhodamine red, tetramethylrhodamine, fluorescein isothiocyanate (FITC), oregon green, Alex Fluoro, carboxyfluorescein (FAM), 6-carboxy-4',5'-dichloro-2',7'-dimethoxyfluorescein (JOE), carboxy-X-rhodamine (ROX), 6-carboxy-2',4,4',5',7, 7'-hexachlorofluorescein (HEX), Texas Red (sulforhodamine 101 acid chloride), 6-carboxy-2',4,7',7-tetrachlorofluorescein (TET), tetramethylrhodamine isothiocyanate (TRITC), carboxytetramethylrhodamine (TAMRA), cyanine dyes, cyanine dicarbocyanine dyes, etc., but is not limited thereto. The cyanine dyes may be Cy3, Cy5, Cy5.5 or Cy7.

The method for detecting a mutant nucleic acid of the present invention detects a mutant gene by measuring a fluorescence signal. Such fluorescence signal measurement may be performed using any microscope capable of detecting single-molecule fluorescence signals, such as total internal reflection fluorescence microscopy (TIRF), confocal microscopy, Epi-fluorescence microscopy, HiLo microscopy, and Line-scanning confocal microscopy, but is not limited thereto.

The method for detecting mutant nucleic acids of the present invention may detect multiple types of mutant nucleic acids simultaneously by amplifying a plurality of targets using multiple types of primer pairs. For example, a second target nucleic acid present in a sample may be amplified using a third primer to which a phosphate group is bound and a fourth primer to which a phosphate group is not bound, together with the first primer and the second primer.

The target nucleic acids may range from several to several dozen.

The method for detecting mutant nucleic acids of the present invention uses probes labeled with different fluorescent molecules for each target nucleic acid to be detected, and may detect a plurality of target nucleic acids by using multi-color imaging.

The method for detecting mutant nucleic acids of the present invention may sequentially detect a plurality of target nucleic acids (second target nucleic acids, third target nucleic acids, etc.) by replacing the probes labeled with fluorescent molecules.

For example, the method for detecting mutant nucleic acids of the present invention may further include the step of contacting a third probe with a nucleic acid strand to which a third chemical substance is bound, for detection of a second target nucleic acid after the step of contacting the first probe.

The third probe contacting step may include contacting a fourth probe targeting a wild-type nucleic acid with a nucleic acid strand bound to a third chemical substance fixed to the detection chip.

The third probe may be a probe that targets a mutant nucleic acid and complementarily binds to it, and may be conjugated to an Argonaute protein, and may be labeled with a third fluorescent molecule.

The fourth probe may be a probe that targets and complementarily binds to a wild-type nucleic acid, and may be conjugated to an Argonaute protein.

The mutant nucleic acid targeted by the third probe is different from the mutant nucleic acid targeted by the first probe, and the wild-type nucleic acid targeted by the fourth probe is different from the wild-type nucleic acid targeted by the second probe.

The third probe may target a plurality of different mutation sites of the same gene, similar to the first probe described above.

The wild-type gene targeted by the fourth probe is the wild-type counterpart of the mutant gene targeted by the third probe.

The detection method of the present invention may detect the presence or absence of mutant types of a plurality of genes in a sample by using probes targeting different genes.

The detection method of the present invention may use a first probe, a third probe, a fifth probe, etc., capable of detecting mutations of each gene according to the number of genes to be detected, and may also use a second probe, a fourth probe, a sixth probe, etc., capable of detecting the wild-types of each gene accordingly.

The mutation detection method of the present invention may further include the step of reverse transcribing RNA, before amplifying the target nucleic acid.

The mutation detection method of the present invention may further include an epigenetic marker detection step of detecting methylation of the nucleic acid by performing bisulfite treatment before amplifying the target nucleic acid.

Hereinafter, the present invention will be described in more detail with examples.

### Examples

### Experimental methods

### 1. Preparation of oigonucleotides

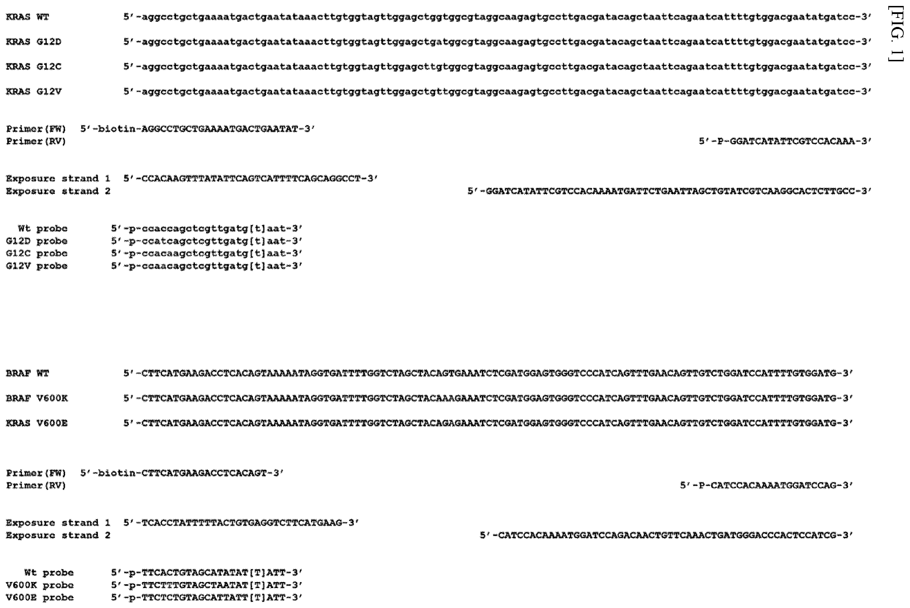

DNA oligomers were purchased from Integrated DNA Technologies (Coralville, IA) or BIONEER (Daejeon, South Korea). The sequence information for the oligonucleotides used in this example is shown in FIG. 1 and Table 1. To label DNA, 0.15 mM amine-modified DNA was incubated with 4.0 mM fluorophore (Cy3, Cy5, or Cy7, mono-reactive NHS-ester) in a labeling buffer (100 mM sodium tetraborate/hydrochloric acid, pH 8.5) for 2 hours. The labeled DNA was purified using a commercial kit (Oligo Clean & Concentrator, Zymo Research) and stored in distilled water at -20°C until use.

**[TABLE 1]**

| Gene | Type | SEQ ID NO |
|---|---|---|
| KRAS | WT | 1 |
| | G12D | 2 |
| | G12C | 3 |
| | G12V | 4 |
| | Primer (FW) | 5 |
| | Primer (RV) | 6 |
| | Exposure strand 1 | 7 |
| | Exposure strand 2 | 8 |
| | Wt probe | 9 |
| | G12D probe | 10 |
| | G12C probe | 11 |
| | G12V probe | 12 |
| BRAF | WT | 13 |
| | V600K | 14 |
| | V600E | 15 |
| | Primer (FW) | 16 |
| | Primer (RV) | 17 |
| | Exposure strand 1 | 18 |
| | Exposure strand 2 | 19 |
| | Wt probe | 20 |
| | V600K probe | 21 |
| | V600E probe | 22 |

### 2. Purification of Tt Ago

*Tt* Ago was expressed as an N-terminal His-tag fusion form. *Tt* Ago purified by Ni-NTA affinity chromatography was further purified by a heat treatment step at 85°C for 15 minutes. After heat treatment, the *Tt* Ago protein was further purified by a size exclusion chromatography step using Superdex S200 26/60 (Cytiva).

### 3. Pretreatment of DNA samples

i) PCR: Synthetic dsDNA samples with different VAFs were amplified using 2 µM 5'-biotinylated forward primer, 2 µM 5'-phosphorylated reverse primer (FIG. 1), and AccuPower Pfu PCR PreMix (BIONEER) at a temperature profile of 94°C, for 3 min, 25 cycles of amplification (94°C for 30 s, 55°C for 30 s, and 72°C for 20 s) and a final extension at 72°C for 1 minute. The amplified dsDNA was then purified using the Expin PCR SV kit (GeneAll Biotechnology). For clinical samples, circulating cell-free nucleic acids were purified from 1 ml of plasma using the QIAamp ccfDNA/RNA kit (Qiagen) according to the manufacturer's protocol prior to PCR. Since the concentration of the extracted cell-free nucleic acids was low, the PCR was performed twice, as described above.
ii) Lambda exonuclease treatment: The 5'-phosphorylated strand of 1.5 µM amplified dsDNA was digested using Lambda exonuclease (M0262, NEB) according to the manufacturer's protocol. The reaction was stopped by adding 10 mM EDTA.
iii) Shielding of non-variant parts: Two exposed strands (FIG. 1) and the remaining 5'-biotinylated ssDNA were mixed in a 2:1 ratio and cooled from 94°C to 4°C.
iv) Surface immobilization: The sample was injected into the detection chamber coated with streptavidin.

### 4. Preparation of Tt Ago-loaded detection and blocking probes

The fluorescently labeled detection probes (0.2 µM each) were incubated with 3 µM *Tt* Ago in assembly buffer (10 mM Tris-HCl (pH 8.0) buffer containing 100 mM NaCl and 5 mM MgCl₂) at 55 °C for 30 minutes. The unlabeled blocking probe (66.6 µM) was incubated with 9.3 µM *Tt* Ago in the same buffer at 55 °C for 30 minutes. Before injection into the detection chamber, the detection and blocking probes were diluted 1/100 and 3/100, respectively, using imaging buffer (20 mM Tris-HCl (pH 8.0) buffer containing 0.5% formamide, 120 mM urea, 135 mM KCl, 1 mM MgCl₂, and an oxygen scavenging system: 4 mg/ml glucose, 2 mg/ml glucose oxidase, 0.08 mg/ml catalase, and 0.4 mg/ml Trolox).

### 5. Single-molecule experiments

Single-molecule experiments were performed using total internal reflection fluorescence (TIRF) microscopy. For TIRF microscopy, the detection chamber was composed of a quartz slide and a glass coverslip. The chamber surface was coated with a 1:20 mixture of biotin-labeled and unlabeled polyethylene glycol. The pretreated sample was fixed to the detection chamber surface through streptavidin-biotin interaction. Then, imaging buffer containing *Tt* Ago-loaded detection and blocking probes was injected into the chamber maintained at 30°C. Cy3, Cy5, and Cy7 were excited by a 532 nm laser (Compass215M, Coherent), a 640 nm laser (Cube640-100C, Coherent), and a 730 nm laser (PhoxX^{®} 730, Omicron), respectively.

Fluorescence signals were collected using a water immersion objective lens (UPlanSApo 60×, Olympus). The collected signals were spectrally separated using dichroic mirrors (635dcxr and 740dcxr, Chroma) and then imaged using an EM-CCD camera (Ixon DV897, Andor). The exposure time of the camera was set to 0.5 s, and three mechanical shutters (LS-3, Uniblitz, Rochester, NY) were alternately switched at a rate of 2 Hz (synchronized with the camera's frame rate) for alternate laser excitation (ALEX). Data were recorded using custom software written in LabView (2009, National Instruments), and data were analyzed using a custom program written in MATLAB (R2013a, MathWorks).

### 6. Statistics and reproducibility

All data were obtained from three independent experiments, and error bars represent standard deviation values.

### Experimental results

### 1. Detection of low-frequency mutations

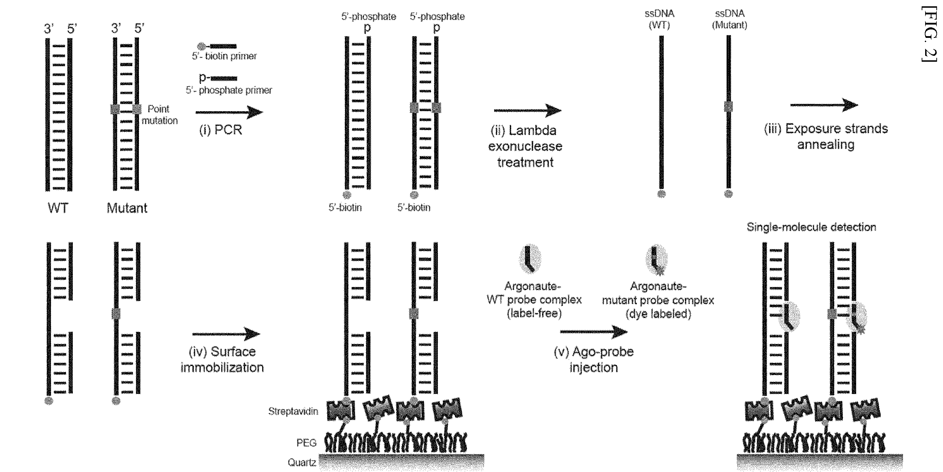

A schematic representation of SIMUL (single-molecule multiplex detection of low-frequency mutations) is shown in FIG. 2. SIMUL begins with a preamplification step using conventional PCR with primers of known sequences. This amplification process targets the genetic region of interest, including both wild-type and mutant ctDNA. The amplified fragments are then treated with λ exonuclease (lambda exonuclease). Since the forward primer is biotinylated at the 5' end and the reverse primer is phosphorylated at the 5' end, λ exonuclease selectively degrades only the antisense strand. After the exonuclease reaction, the sense strand is annealed to the exposed strand (a complementary oligomer that is unlabeled at both the 5' and 3' ends), thereby exposing only the hotspot target region of the single-stranded DNA.

After this pretreatment step, the annealed DNA is fixed to the surface of the detection chamber via streptavidin-biotin conjugation. A fluorophore-labeled detection probe, designed to specifically bind and detect the target ctDNA, is then introduced into the detection chamber, and transient binding events are visualized using TIRF microscopy. To monitor binding of the detection probe, Ago-FISH technology is exclusively used, in which the detection probe is loaded onto the Argonaute protein from *Thermus thermophilus (Tt* Ago) to accelerate the target binding process. An unlabeled blocking probe pre-loaded with *Tt* Ago is also added to the chamber together with the detection probe. The blocking probe is fully complementary to the wild-type DNA and serves to minimize or prevent binding of the detection probe to the wild-type sequence.

To evaluate the ctDNA detection capability, 114-bp synthetic double-stranded DNA (dsDNA) representing the wild-type and G12V mutation of the human KRAS proto-oncogene gene was prepared (FIG. 3). G12V is a notable oncogenic mutation frequently found in non-small cell lung cancer (NSCLC) and is more prevalent among smokers. This mutation serves as a key prognostic biomarker, indicating poor overall survival and a higher relapse rate.

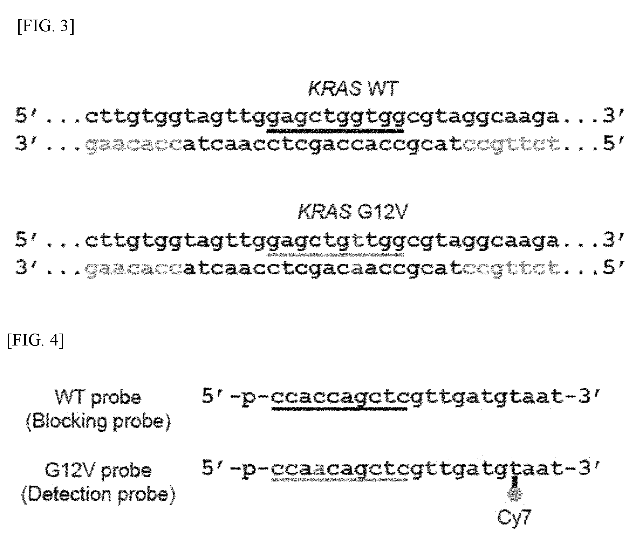

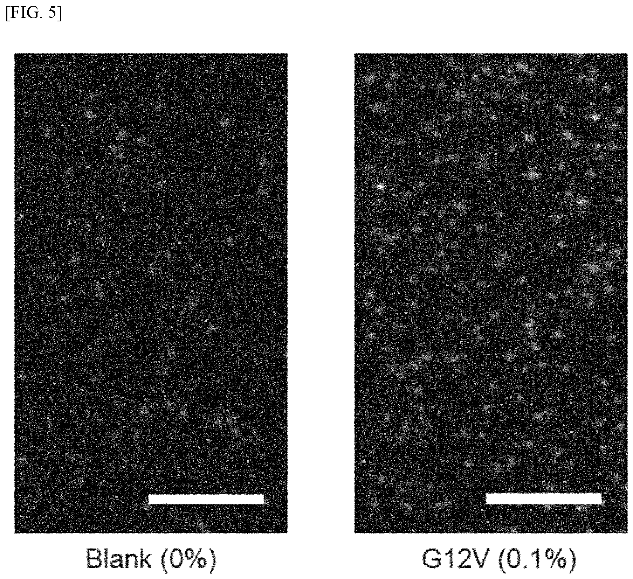

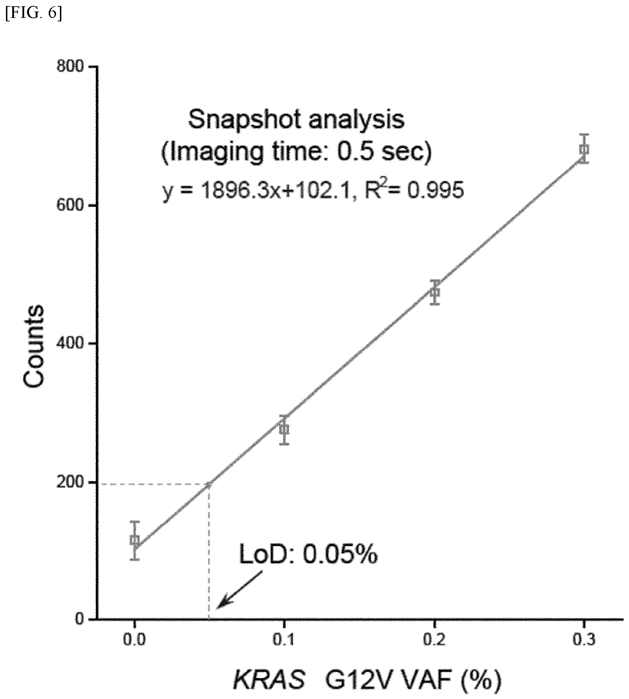

After pretreatment and surface immobilization of the blank (0%) and 0.1% VAF samples of the KRAS G12V mutant, the Cy7-labeled detection probe was injected into the detection chamber together with the unlabeled blocking probe complementary to wild-type KRAS (FIG. 4). Single-frame snapshots of the Cy7 fluorescence images were then captured at a 0.5-second exposure. Significantly more fluorescent spots were detected in the 0.1% VAF sample than in the blank sample (FIG. 5). It was also examined how the number of Cy7 spots changed as the VAF of G12V increased in the range of 0 to 0.3%. A strong linear correlation was observed between the number of single-molecule spots and VAF over the entire range of variation. The limit of detection (LoD), defined as the mean of the blank sample plus three standard deviations (3σ), was measured to be 0.05% VAF (FIG. 6).

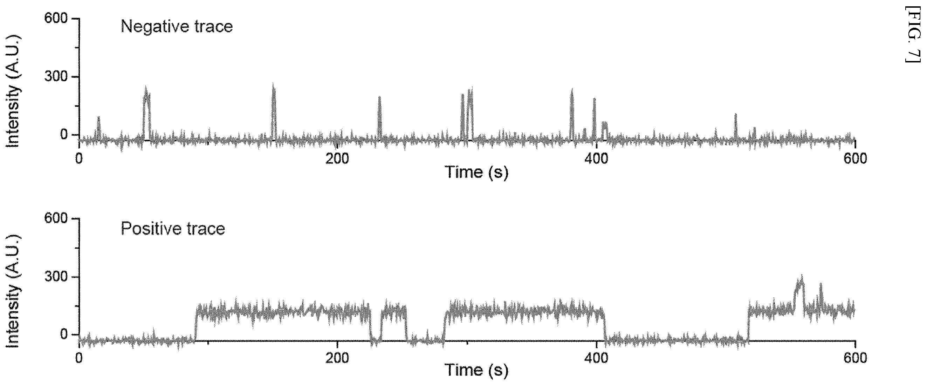

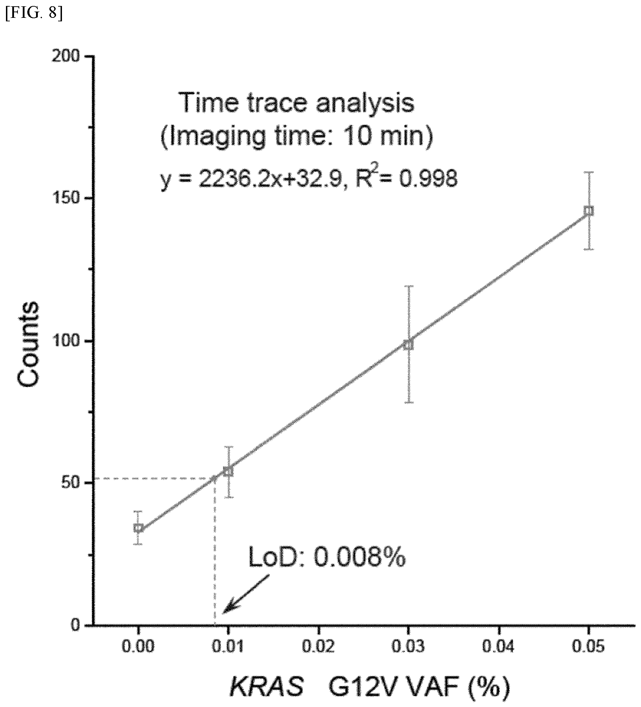

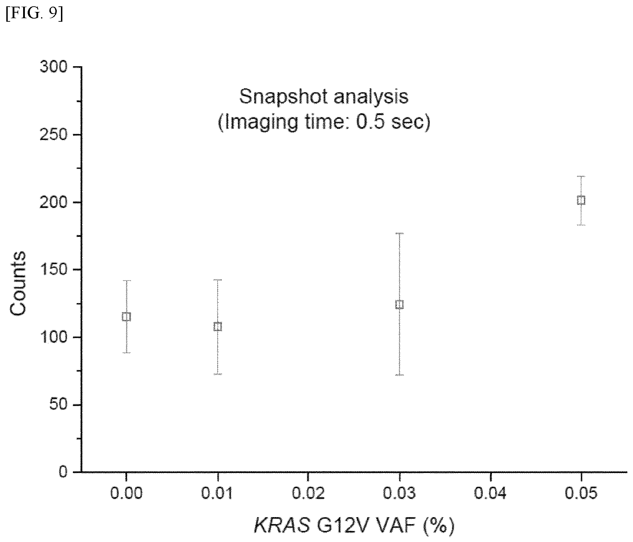

The ability of Ago-FISH to distinguish single nucleotide differences has been reported to be significantly improved by analyzing the binding/dissociation kinetics of the detection probe for on-target and off-target sites. To distinguish on-target and off-target binding, a 20% duty cycle (representing the percentage of observation time during which the detection probe remains bound to the target DNA) was selected as a threshold for identifying KRAS G12V mutations (FIG. 7). This approach allowed an investigation of how the number of mutations changed as the VAF of the mutations increased from 0 to 0.05%. A strong linear relationship was observed between the number of spots and the VAF, with the LoD measured at 0.008% VAF (FIG. 8). This similar linearity was not observed when single-frame fluorescence images were used for data analysis (FIG. 9).

### 2. Multiplexing capabilities of SIMUL

In principle, multiple target ctDNAs may be detected simultaneously using single-molecule multi-color imaging. To demonstrate this capability of SIMUL, dsDNA was utilized. Wild-type KRAS and G12C, G12D, and G12V mutations, and their corresponding probes were used (Table 2). For single-molecule imaging, the detection probes for G12D, G12C, and G12V were labeled with Cy3, Cy5, and Cy7, respectively. In contrast, the blocking probe complementary to wild-type KRAS was left unlabeled to minimize the off-target binding of the labeled detection probes to wild-type KRAS.

**[TABLE 2]**

| KRAS synthetic dsDNA | | SEQ ID NO |
|---|---|---|
| WT | 5'->3' | 23 |
| | 3'->5' | 24 |
| G12C | 5'->3' | 25 |
| | 3'->5' | 26 |
| G12D | 5'->3' | 27 |
| | 3'->5' | 28 |
| G12V | 5'->3' | 29 |
| | 3'->5' | 30 |

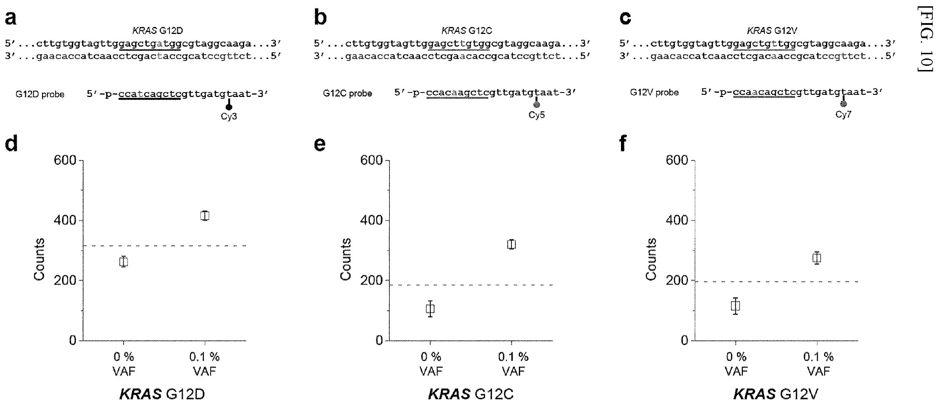

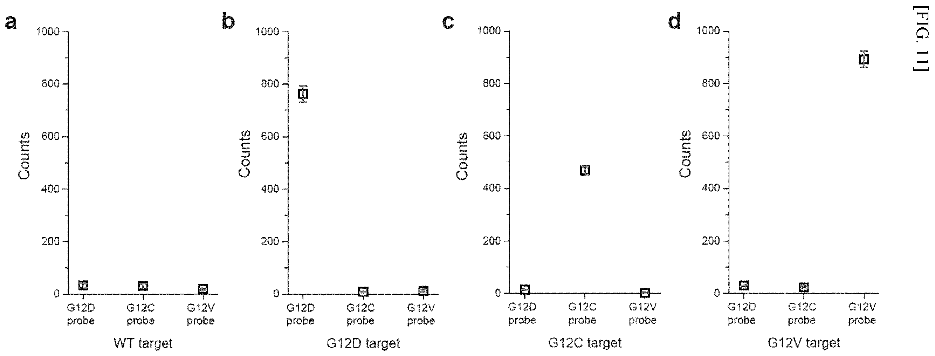

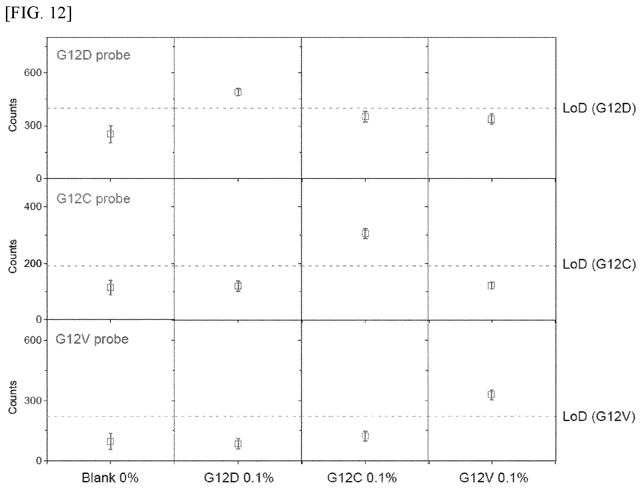

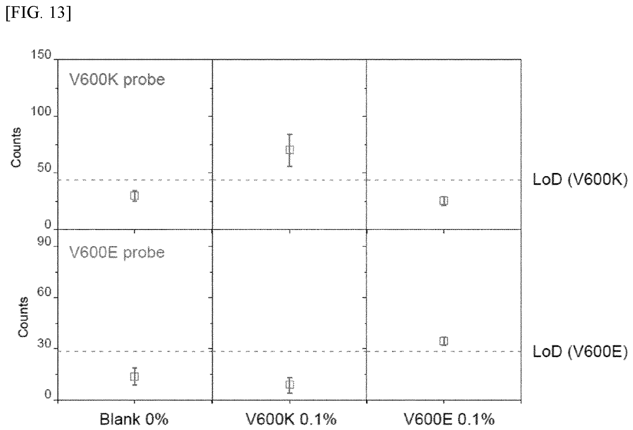

The sensitivity (FIG. 10) and orthogonality (FIG. 11) of the detection probes were independently validated. After preparing blank and 0.1% VAF samples of KRAS mutations, single-frame spot counts of Cy3, Cy5, and Cy7 fluorescence were quantified in the presence of all probes. The number of spots in each fluorescence channel exceeded the LoD only when the corresponding mutation was present in the test sample (FIG. 12). The multiplexing capability of SIMUL was further confirmed using wild-type BRAF and the V600E and V600K mutations, based on multi-color imaging of other ctDNA targets (Table 3 and FIG. 13).

**[TABLE 3]**

| BRAF synthetic dsDNA | | SEQ ID NO |
|---|---|---|
| WT | 5'->3' | 31 |
| | 3'->5' | 32 |
| V600K | 5'->3' | 33 |
| | 3'->5' | 34 |
| V600E | 5'->3' | 35 |
| | 3'->5' | 36 |

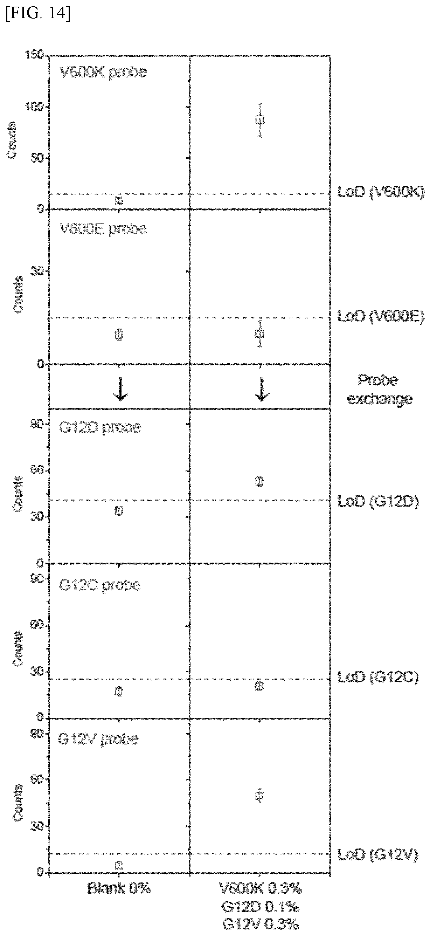

Multi-color imaging-based multiplexing may be effectively scaled up by incorporating sequential probe exchange. To demonstrate the scalability of SIMUL, BRAF and KRAS DNA were simultaneously amplified in a single reaction tube, and the amplified mixture was fixed to a single detection chip and imaged. The mixture in the experiment consisted of 0.3% BRAF V600K mutation, 0.1% KRAS G12D mutation, and 0.3% KRAS G12V mutation. By sequentially applying BRAF and KRAS probes, it was observed that the number of spots exceeded the LoD only for the established mutation, while falling below the LoD for the other mutations. The scalability of SIMUL was successfully demonstrated using a different mixture containing 0.1% BRAF V600K mutation, 0.3% BRAF V600E mutation, and 0.3% KRAS G12V mutation (FIG. 14).

### 3. Detection of ctDNA in plasma from cancer patients

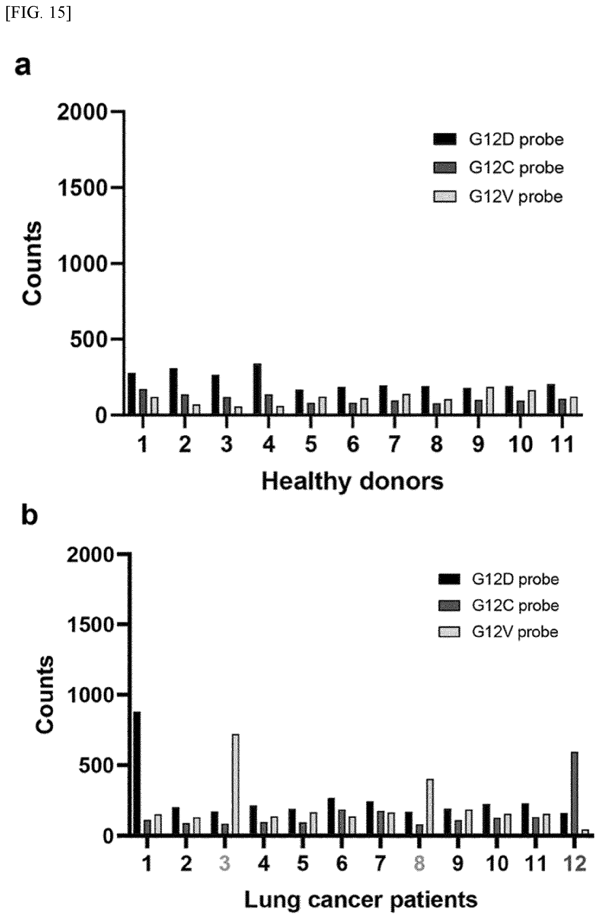

After characterizing the detection speed, measurement sensitivity, and multiplexing capabilities of SIMUL using dsDNA, this technology was used to identify ctDNA in plasma from lung cancer patients. To perform this experiment, circulating cell-free DNA (ccfDNA) was isolated from 1 ml of human plasma, followed by the specific amplification of KRAS ccfDNAs. The amplified products were then fixed to a single detection chip, and KRAS G12C, G12D, and G12V mutations were detected simultaneously (FIG. 15).

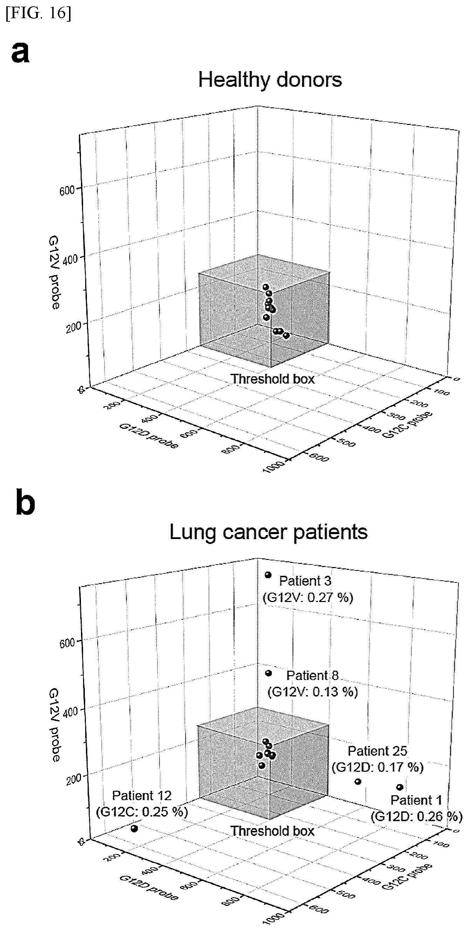

In plasma from 11 healthy control donors, the number of mutant fluorescent spots remained below the LoD in all cases (FIG. 16a). In contrast, in plasma from 12 lung cancer patients, four positive signals were detected (one KRAS G12C, one KRAS G12D, and two KRAS G12V), thereby confirming the presence of mutant VAFs in the plasma (FIG. 16b).

## Claims

1. A method for detecting a mutant nucleic acid, comprising the steps of:
amplifying a first target nucleic acid present in a sample using a pair of primers comprising a first primer to which a phosphate group is bound and a second primer to which the phosphate group is not bound, to obtain an amplified product;
treating the amplified product with a nuclease that specifically binds to the phosphate group to obtain a single-stranded first target nucleic acid to which the phosphate group is not bound; and
fixing the single-stranded first target nucleic acid to a surface of a detection chip and then contacting it with a first probe labeled with a first fluorescent molecule.

2. The method for detecting a mutant nucleic acid according to claim 1, further comprising the step of detecting whether the first probe is bound to a mutation site of the single-stranded first target nucleic acid.

3. The method for detecting a mutant nucleic acid according to claim 1, further comprising the step of converting the remaining region of the single-stranded first target nucleic acid, excluding the binding region of the first probe, into a double-stranded region, before fixing the single-stranded first target nucleic acid to the surface of the detection chip.

4. The method for detecting a mutant nucleic acid according to claim 1, wherein the second primer to which the phosphate group is not bound is a second primer bound to biotin or digoxigenin.

5. The method for detecting a mutant nucleic acid according to claim 1, wherein the single-stranded first target nucleic acid is fixed to the surface of the detection chip by a complementary nucleic acid.

6. The method for detecting a mutant nucleic acid according to claim 5, wherein the complementary nucleic acid used for fixing to the surface of the detection chip is a nucleic acid bound to biotin or digoxigenin.

7. The method for detecting a mutant nucleic acid according to claim 1, wherein the sample is any one selected from the group consisting of blood, plasma, serum, urine, feces, cerebrospinal fluid, lung lavage fluid, pleural effusion, ascites, saliva, sputum, body fluid, tissue, and cells isolated from an individual.

8. The method for detecting a mutant nucleic acid according to claim 1, wherein the nucleic acid is DNA or RNA.

9. The method for detecting a mutant nucleic acid according to claim 1, wherein the nuclease is lambda exonuclease.

10. The method for detecting a mutant nucleic acid according to claim 1, wherein the surface of the detection chip is coated with avidin or anti-digoxigenin.

11. The method for detecting a mutant nucleic acid according to claim 1, further comprising the step of reverse transcribing RNA before amplifying the first target nucleic acid.

12. The method for detecting a mutant nucleic acid according to claim 1, further comprising the step of pretreating the nucleic acid to detect methylation thereof before amplifying the first target nucleic acid.

13. The method for detecting a mutant nucleic acid according to claim 1, wherein the first probe is conjugated to an Argonaute protein.

14. The method for detecting a mutant nucleic acid according to claim 2, wherein the detection is performed by measuring a FRET signal.

15. The method for detecting a mutant nucleic acid according to claim 1, further comprising the step of amplifying a second target nucleic acid present in the sample using a pair of primers comprising a third primer to which a phosphate group is bound and a fourth primer to which the phosphate group is not bound.

16. The method for detecting a mutant nucleic acid according to claim 15, further comprising the step of fixing a single-stranded second target nucleic acid to which the phosphate group is not bound to the surface of the detection chip, and then contacting it with a third probe labeled with a third fluorescent molecule to perform multi-color imaging.

17. The method for detecting a mutant nucleic acid according to claim 1, further comprising the step of treating a second probe that complementarily binds to a wild-type nucleic acid and is not fluorescently labeled, together with the first probe.
